# EUROPEAN PATENT APPLICATION

(11) **EP 3 617 143 A1**
(43) Date of publication of application: **04.03.2020**
(21) Application number: 18191359.1
(22) Date of filing: 29.08.2018
(51) Int. Cl.: C01B 33/00, C05D 9/02, C05F 11/08, C12N 1/14, C12P 3/00

(54) **PROCESS FOR OBTAINING STABLE SUSPENSIONS OF SELENIUM AND SILICON NANOPARTICLES**

(30) Priority: 28.08.2018 RO 201800616
(71) Applicant: Institutul National de Cercetare-Dezvoltare Pentru Chimie si Petrochimie-Icehim, 060021 Bucharest (RO)
(72) Inventor: OANCEA, Florin, 062082 Bucuresti (RO); CONSTANTINESCU-ARUXANDREI, Diana, 030306 Bucuresti (RO); CALIN, Mariana, 060962 Bucuresti (RO); RAUT, Iuliana, 032768 Bucuresti (RO)
(74) Representative: Teodorescu, Mihaela

(57) **Abstract**

The invention relates to a process for preparing stable suspensions of associates zerovalent selenium and silica nanoparticles, intended for application as plant biostimulants, to increase resistance to abiotic stress factors, to enhance plant nutrients uptake and use and to improve yield quality. Process according to the invention includes the following steps: cultivation of microbial strains highly producing amphiphile proteins with affinity for carbohydrates, on a mediums that promote the induction of those genes, separation of the plant biostimulants fungal mycelium and adding over biosilica rich plant material together with sterile minimal mineral medium, incubation, together with amphiphile proteins recovered from the original medium, removal of the mycelium and plant material, aseptic adding of a 10 mM sodium selenite solution, in Supernatant containing spores, excess amphiphile proteins, silica nanoparticles, incubation for the formation of the selenium nanoparticles, followed by tangential ultrafiltration separation of the newly formed mycelium, concentration of nanoparticles suspension resulted as permeate through tangential ultrafiltration, followed by its sterilization by ultrafiltration.

## Description

This invention relates to a process for preparing stable suspensions of associated zerovalent selenium and silica nanoparticles, intended for application as plant biostimulants, to increase resistance to abiotic stress factors, to enhance plant nutrients uptake and used to improve yield quality.

There are several known processes for the preparation of stable suspensions of associated selenium and silica nanoparticles. Such suspensions of nanoparticles have various applications. Both elemental (zerovalent) selenium nanoparticles and silica nanoparticles (silicon dioxide, especially the hydrated and amorphous form, SiO₂ *x*nH₂O), due to their high surface - volume ratio, function as a reservoir for the gradual release of bioactive chemical species, selenide, selenite and/or selenate ions (Skalickova et al. 2017, Nutrition, 33:83-90), respectively orthosilicic acid (Quignard et al. 2017, Colloids and Surfaces B: Biointerfaces, 155:530-537). Such species of selenium and bioactive silicon act on cultivated plants as inorganic plant biostimulants (du Jardin, 2015, Scientia Horticulturae, 196:3-14).

Silicon is not considered as an essential element for plants, but only as having beneficial effects (Yan et al 2018, Journal of Integrative Agriculture, 17:60345-7). Studies have shown that silicon has all the characteristics of the plant biostimulants (Savvas And Ntatsi, 2015, Scientia Horticulturae, 196: 66-81). Soluble silicon is one of the few elicitors that induces the different metabolic pathways involved in the plant defense response, in an equilibrated manner (Van Bockhaven et al. 2013, Journal of Experimental Botany, 64: 1281-1293). The action of soluble silicon is not limited only to the orchestration of the metabolic pathways involved in the protection of plants against the attack of pathogens and pests, but it also has effects on increasing the efficiency of the use of nutrients, on the decrease in toxicity of potentially toxic elements/ heavy metals, and on the limitation of the effects of hydric stress (salinity, drought) and of the thermic stress (frost, high temperatures) (Frew et al. 2018, Annals Of Botany, 121: 1265 - 1273).

The most accessible silicon for plants is that of biological origin, specially the silicon from plant material. From such bio-structures, the release of soluble silicon species is not accompanied by the release of contra-ions, such as aluminum, iron, or calcium.

In the case of siliceous rocks or siliceous amendments, e.g. blast furnace slag, the additional element present, respectively iron (Fe) and aluminum (Al), reduces silicon bioavailability. Silicic acid, which is a molecular species partly mobile in soil solution, is precipitated in the form of secondary clays in contact with immobile ions (in soil) Fe³ + and Al³+ (Sommer et al. 2006, Journal of Plant Nutrition and Soil Science, 169:310-329).

Silicic acid salts generate a high pH by hydrolysis, silicic acid being a very weak acid. The high pH of these salts limits their application to acidic soil which needs to be amended to neutral pH. Also, the salts of the orthosilicic acid are effective only for a short term, having the disadvantage of generating too high concentrations of orthosilicic acid by solubilization in the soil solution, up to the concentration limit of 2 mM, for the soluble silicon to be up-taken by the roots (Tubana et al. 2016. Soil Science, 181: 393-411). At this concentration limit silicic acid generates reactions of poly/oligo-condensation, with the formation of molecular species of silicon with lower bioavailability (Spinde et al. 2011, Chemistry of Materials, 23:4676-4687).

Compared to other forms of silicon, silica nanoparticles are more effective as plant biostimulants, especially in the case of foliar application (Laane, 2018, Plants, 7: 45). A series of processes intended to prepare biogenic nanoparticles are known. Patent US9403688 B1 presents a process to prepare biogenic silica nanoparticles, which includes the following steps: pre-treating seed hulls of a biogenic source with an acid to form acid-treated seed hulls; placing the acid-treated seed hulls in an autoclave, for about 2 hours, at temperature greater than 100°C, under a fixed pressure; isolating the seed hulls; washing the seed hulls with water; air drying the seed hulls; calcining the seed hulls at temperature between 500°C and 700° C for at least one hour in a furnace to produce biogenic silica nanoparticles. The resulted biogenic silica nanoparticles are amorphous and biocompatible, possessing particle sizes in the range of 25-75 nm.

The WO2017042011 patent application refers to a process for the extraction of silica, comprising the following steps: a) fractionating the lignocellulosic plant material in the presence of an acid solution, to obtain a solid fraction comprising cellulose, b) extracting the silica from the solid fraction obtained in step a) with a basic solution, at pH between 10 and 13 and at temperature between 70°C and 90°C, to obtain a liquid phase comprising silica, and a solid phase, c) separating the liquid phase and the solid phase which are obtained in step b), d) precipitating the silica which is found in the liquid phase, at pH between 5 and 6.

The above procedures are chemical processes and have the disadvantage of high consumptions of acids and bases, which impact on the environment during their production and their use. Biotechnological "green" processes, with a high eco-efficiency, would be more useful in producing biogenic silica nanoparticles.

A biological process to convert some plant substrates, with a high level of silica structures formed in the cell wall (e.g. rice husk) into silica nanoparticles was published (Zielonka et al. 2018, Fungal Biology, 122: 333-344). However, the strain used, *Aspergillus parasiticus* NRRL 2999, is a standard strain to produce aflatoxins (Rasooli And Abyaneh, 2004, Food Control, 15: 479-483), and therefore this process has only a theoretical significance, to demonstrate the possibility of biological conversion, and is not usable in practice.

Selenium promotes plant development (Hartikainen And Xue, 1999, Journal of Environmental Quality, 28: 1372-1375; Xue et al. 2001, Plant and Soil, 237: 55-61), has a role in plant protection against plant pathogen agents (Hanson et al. 2003, New Phytologist, 159: 461-469), and against abiotic stress factors, including heavy metals (Feng et al. 2013, Environmental and Experimental Botany 87:58-68) and drought (Ahmad et al. 2016, Journal of the Science of Food and Agriculture 96:372-380).

Inorganic selenium compounds are used for the treatment of plants. Inorganic selenium species have a toxicity that decreases in the order of Selenate > Selenite, selenide > elemental / zerovalent Selenium (Nuttall, 2006, Annals of Clinical & Laboratory Science 36:409-420). While selenate has very high toxicity in rats, higher than potassium cyanide, selenium zerovalent has a two-order lower-size toxicity (Olson, 1986, Journal of the American College of toxicology 5:45-70). Zerovalent selenium nanoparticles (SeNPs) have shown even lower toxicity than that of elemental selenium (Shakibaie et al. 2013, Pharmaceutical Biology 51:58-63). At the same time, the efficacy of selenium nanoparticles (SeNPs) in the induction of seleno-enzymes is comparable to that of the most effective form of organic selenium, Seleno-methyl-Seleno-Cysteine (SeMeSeCys) (Zhang et al. 2008, Toxicological Sciences 101:22-31). The latter has similar toxicity to that of selenite and is very difficult to prepare, by a difficult and low-yield organic synthesis (Iwaoka et al. 2016, Proceedings of the National Academy of Sciences, Section A. 86:499-509).

The chemical production of selenium nanoparticles involves the use of concentrated mineral acids (Stroyuk et al. 2008, Colloids and Surfaces A: Physicochemical and Engineering layouts 320:169-174) or of toxic reagents such as hydrazine (Mishra et al. 2005, The Journal of Physical Chemistry B109:12718-12723) or ionic liquids (Near et al. 2010, Materials Research Bulletin 45:668-671).

Biosynthesis of selenium nanoparticles is carried out in mild conditions (Wadhwani et al. 2016, Applied Microbiologist and Biotechnology 100:2555-2566). In general, biologically synthesized SeNPs are up to ten times less toxic than chemically synthesized nanoparticles (Mal et al. 2017, Nanotoxicology 11:87-97).

Preparation of selenium nanoparticles by biosynthesis has obvious advantages. Patent Application CN 107881127 (A) discloses a strain of *Bacillus amyloliquefaciens*, Lxz-41, deposited under M2016578 number at China Center for Type Culture Collection (CCTCC), and a method for controllable preparation of nano-selenium by using this strain. The biosynthesis process of selenium nanoparticles involves the following steps: cultivation on a glucose and enzymatic peptone based medium, that also contains a surfactant, adding a selenium salt, from the beginning or through the continuous feeding, cell harvesting, ultrasonication of the bacteria and separation of endonanoparticles from bacterial cells. Patent application CN105199979 (A) discloses a strain of *Bacillus thuringiensis* YLX-4, isolated from percolating waters from a selenium mine (Enshi, Hubei Province of China), deposited with M2013674 number at China Center for Type Culture Collection (CCTCC). The strain has the typical characteristics of seleno-bacteria and was proposed for several applications, including nano-selenium production.

Concomitant application of selenium and silicon to plants determines synergistic effects in protection from abiotic stress caused by drought (Emam et al. 2014, Australian Journal of Crop Science 8:596), salinity (Sattar et al. 2017, Russian Journal of Plant Physiology 64:341-348) and heavy metals (Gao et al. 2018, Science of The Total Environment, 631: 1100-1108).

To harness this synergism between selenium and silica the Patent US 9,919,978 B2 describes a nanosol of silica in which nano-selenium, obtained by reducing selenious acid and/or its salts with ascorbic acid, with Glutathione or with reducing carbohydrates, is doped. The hydrophobic selenium nanoparticles of maximum 50 nm are maintained in the silica nanosol by emulsification with polyvinylpyrrolidone, polyvinyl alcohol, or polyoxyethylenesorbitan monolaurate - Tween 20. The resulting product reduces the absorption of cadmium by rice plants and concomitantly, agronomic biofortifies the rice culture with selenium.

The preparation process of a silica nanosol in which the nano-selenium is doped, presented in the patent US 9,919, 978 B2 implies seven steps in which strong acids and bases are used, including toxic ammonia solutions. The disadvantage of this product that associates selenium and silica nanoparticles is common with that of other chemically synthesized nanoparticles. Such chemically synthetized nanoparticles have a low reproducibility of the biological activity, because they randomly form a biocorona, from proteins and/or other biomolecules, during the interaction with biological systems (Guerrini, 2018, Materials, 11: 1154). The effects on plants are significantly mediated by this corona (Me et al. 2018, Annual Review of food Science and Technology 9: 129-153). The control on the corona of nanoparticles is essential for their biological activity (Ke et al. 2017, ACS Nano 11:11773-11776).

Nanoparticles produced by biosynthesis / assisted biosynthesis have the advantage of forming a corona with higher stability from the (bio)synthesis stage. This type of corona formed from the biosynthesis / bio-assisted synthesis step amplifies the properties of nanoparticles - for example the nano-selenium formed by bio-assisted synthesis in the culture media of fungi from the genus *Trichoderma* enhances the disease suppressive ability of *Trichoderma* against downy mildew disease caused by *Sclerospora graminicola* in pearl millet (Nandini et al. 2017, Scientific reports 7: 2612). An additional advantage of the synthesis processes of nanoparticles in the biological environments is that they are "green" processes that do not involve high temperatures or extreme pH.

In the case of selenium nanoparticle suspensions associated with silica nanoparticles, the stability is affected by the different character of these two types of nanoparticles, the strong hydrophobicity of nano-zerovalent selenium and the strong hydrophilicity of (hydrated) silica. The use of surfactants to stabilize the suspensions of nanoparticles presents the risk of determining changes in the biocorona composition (Müller et al. 2018, Biomacromolecules 19:2657 - 2664).

The technical problem which this invention solves is to achieve compatibility between the hydrophobic selenium nanoparticles and the hydrophilic silica nanoparticles and to increase suspension stability. The technical solution is to develop a biosynthesis process of stable suspensions of associated selenium and silica nanoparticles, by using strains having plant biostimulant activity and which produce large amounts of amphiphile proteins, with affinity for carbohydrates, such as microbial expansins, cerato-platanins, swolenins, cellulases. Such amphiphile proteins disrupt the hydrogen bonds of the lignocellulose material structure (Cosgrove, 2017. Annual Review of Microbiology, 71: 479-497; Bacelli, 2015, Frontiers in Plant Science 5:769), due to their small molecular mass, their amphiphilic character (Bonazza et al. 2015, Soft Matter 11: 1723-1732) and flexible structure, allowing the independent movement of hydrophobic and hydrophilic parts (Sunde et al. 2017, Annual Review of Biochemistry 86: 585-608).

The process for preparing stable and associated suspensions of zerovalent selenium and silica nanoparticles, according to the present invention includes the following steps:
✔ Cultivation of microbial strains having plant biostimulant action on plants, and highly producing amphiphile proteins with affinity for carbohydrates, on a medium that promotes the induction of the amphiphile proteins genes, at 28-30°C, on a rotary shaker, 70 rpm, for 5 days;
Axenic separation of the plant biostimulant fungal mycelium from the supernatant, by centrifugation, at 2500 x g;
✔ Moistening of the biosilica rich plant material with ultrapure or double-distilled water, in the proportion of 1 g plant material to 1 ml pure or double-distilled water;
✔ Sterilization of moistened biosilica rich plant material, by three repeated cycles, heating at 72 to 75°C for 25-30 min and cooling at room temperature for 6 hours;
✔ Aseptically adding the wet mycelium of the plant biostimulant microorganisms over the moistened and sterilized biosilica rich plant material, in the ratio of 1 g wet mycelium to 5 g plant material;
✔ Pouring a sterile minimal mineral medium over the mycelium - plant material mixture, in a ratio of 80 ml sterile minimal mineral medium to 20 grams of mycelium - plant material mixture;
✔ Preparation of a protein solution, by protein purification from the supernatant separated at the second step, concentration and sterilization by filtration, to be added over the mycelium - plant material mixture, in order to increase the concentration of the amphiphile proteins;
✔ Incubation of the mixture mycelium - plant material - protein solution at the temperature of 28 - 30°C, on a rotary shaker, 70 rpm, for 5-7 days;
✔ Removal of the mycelium and plant material by centrifugation at 1000 x g or by filtration on membranes with pores of minimum 0.45 µm;
✔ Aseptically adding 10 mM sodium selenite solution in the supernatant obtained in the previous step and containing spores from mycelium, amphiphile proteins, silica nanoparticles associated with carbohydrates, in a ratio of 10 ml sodium selenite solution to 90 ml supernatant;
✔ Incubation of the medium containing spores from mycelium, amphiphile proteins, silica nanoparticles, and sodium selenite, at 28-30°C, on a rotary shaker, 70 rpm, for 5-7 days, followed by separation of the newly formed mycelium, by tangential ultrafiltration on a membrane of 0.45 µm;
✔ Concentration by tangential ultrafiltration of the nanoparticles suspension resulted, as permeate from the previous tangential ultrafiltration, followed by its sterilization.

The strains of microorganisms with plant biostimulant action, high producers of amphiphile proteins with affinity for carbohydrates are selected between the strains of Hypocreaceae family, e.g. *Trichoderma harzianum* Td50B, *Trichoderma asperellum* Td36b, *Trichoderma* spp. T27.

The medium which promotes the expression of amphiphile proteins with affinity for carbohydrates has the following composition: 10 g/L whey powder with min. 12% protein and min. 72% lactose, 0.68 g/L KH₂PO4, 0.87 g/L K₂HPO4, 1.2 g/L (NH₄)₂SO4, 0.2 g/L KCI, 0.2 g/L CaCl₂, 0.2 g/L MgSO₄.7H₂O, 2 mg/L FeSO₄.7H₂O, 2 mg/L MnSO₄.7H₂O and 2 mg/L ZnSO₄.7H₂O.

Plant material with high silicon content includes rice husk, *Pleurotus* spent substrate, brewer's spent grain, as well as their mixtures.

The used mineral minimal medium is known from the state-of-the art and has the following composition: KH₂PO₄ 1.0 g/L, K₂HPO₄ 1.0 g/L, NaCl 0.5 g/L, NH₄NO₃ 1.0 g/L, MgSO₄·7H₂O 0.2 g/L, CaCl₂ 20 mg/L and FeCl₃·6H₂O 5 mg/L.

The purification of the amphiphile proteins produced in the initial media is done by hydrophobic affinity chromatography on a hydrophobic column, and the concentration of proteins is done by ultrafiltration. The resulted protein solution has a concentration in the range 8-10 mg/ml.

The procedure according to the invention presents the following advantages:
✔ Stimulates the expression of genes for cerato-platanins and cellulases, microbial expansins, swolenins, by cultivation on mediums containing lactose and proteins with hydrophobic amino acids and amino acids with branched chains;
✔ Releases the nano-silica particles from the plant material through the combined action of cellulases and amphiphilic proteins - microbial expansins, cerato-platanins, or swolenins - on plant material;
✔ Biologically generates selenium nanoparticles by the reductive detoxification of selenite by the metabolism of the microorganisms with plant biostimulant activity;
✔ Promotes the formation of selenium nanoparticles with a biocorona, consisting mostly of amphiphilic proteins, microbial expansins, cerato-platanins, or swolenins, newly formed or pre-formed in the original medium recovered by centrifugation.
✔ Determines the compatible association between the hydrophobic selenium nanoparticles and the hydrophilic silica nanoparticles associated with carbohydrates, through the action of amphiphilic proteins, microbial expansins, cerato-platanins, or swolenins, proteins with high flexibility and allowing the free movement of the hydrophilic and hydrophobic part.
✔ Potentiates the biostimulant activity on cultivated plants by associating nanoselenium with nanosilica and with metabolites produced by microbial biostimulants.

Further, examples of invention embodiments are presented, which illustrate it, without limiting it.

*Example 1*. 2 liters of medium (medium A), which promotes the expression of cellulases and microbial expansins, cerato-platanins, and/or swolenins, with the following composition, are prepared: 10 g/L whey powder with min. 12% protein and min. 72% lactose, 0.68 g/L KH₂PO₄, 0.87 g/L K₂HPO4, 1.2 g/L (NH₄)₂SO₄, 0.2 g/L KCI, 0.2 g/L CaCl₂, 0.2 g/L MgSO₄.7H₂O, 2 mg/L FeSO₄.7H₂O, 2 mg/L MnSO₄.7H₂O and 2 mg/L ZnSO₄.7H₂O in a glass flask of 2.5 liters. The medium is distributed in 10 Erlenmeyer flasks of 1 liter, 200 mL in each flask. Flasks are covered with cotton plugs, sterilized by autoclavation at 121 °C for 20 min and cooled. Each flask is inoculated with 2 ml suspension 10⁹ propagule/ml from the strain *Trichoderma harzianum* Td50b, deposited with deposit number NCAIM (P) F 001412 to National Collection of Agricultural and Industrial Microorganisms, University Corvinus, Budapest, Hungary. The inoculated flasks are incubated at the temperature of 28°C, on a rotary shaker, at 70 rpm, for 5 days. After 5 days the mycelium with the culture media is axenically transferred in 4 sterile centrifuge tubes of 500 mL. The tubes are centrifuged in a centrifuge Eppendorf 5810 (Eppendorf, Hamburg, Germany), with swinging rotor A-4-81, in which the 4 tubes are introduced, equilibrated 2 by 2. The tubes are centrifuged at the speed of 3525 rpm, corresponding, on the swinging rotor A-4-81, with a radius of 18 cm, to a relative centrifugal force of 2500 *x* g.

In a 5 liters flat bottom glass flask 500 g of rice husk are brought, over which 500 mL of ultrapure water (MiliQ) are slowly poured on a glass rod. The flask is left at room temperature for 2 hours and the mixture is stirred every 10 minutes. The flask is covered with a cotton plug, is heated on a thermostated water bath (Lab Companion 37 L, Cole Parmer, Vernon US), up to 75°C, where it is maintained for 25 minutes. It is cooled at room temperature for 12 hours and then the heating/ maintenanc/ cooling cycles are repeated twice. After these 3 heating / cooling cycles (tyndallization), which destroy the vegetative forms of microorganisms, including those that form from heat resistant endospores during the cooling/ room temperature maintenance process, the flask is inoculated with fungal mycelium.

Over the 500 g of moistened and sterilized rice husk, 100 g of fungal mycelium, fresh biomass of *Trichoderma harzianum* Td50b are added aseptically. 2.4 liters of minimal mineral medium with the following composition are prepared: KH₂PO₄ 1.0 g/L, K₂HPO₄ 1.0 g/L, NaCl 0.5 g/L, NH4NO₃ 1.0 g/L, MgSO₄.7H₂O 0.2 g/L, CaCl₂ 20 mg/L and FeCl₃.6H₂O 5 mg/L. The medium is sterilized by autoclaving for 20 min at 121 °C, it is cooled and then it is added over the moistened and sterilized vegetal material and mycelium.

From the initial supernatant of the induction medium the amphiphile proteins are separated and purified by hydrophobic affinity chromatography. The approximately 1.8-liter supernatant are applied gradually on a column of hydrophobized agarose, Phenyl Sepharose® 6 Fast Flow (45 × 10 cm, Ge Healthcare, Chicago, IL, USA), equilibrated with 100 mM Tris/HCI, pH 7.5, containing 2 M ammonium sulphate. Theamphiphile proteins that include the cerato-platanins of interest are eluted with water, by a linear gradient, starting from the equilibration buffer till 2 mM Tris/HCI pH 7.5. The fractions containing the amphyphile proteins are concentrated by tangential ultrafiltration, on a tangential ultrafiltration system Prostak (Merck Group, Darmstadt, Germany), fitted with a membrane Ultracel (Merck Group) of regenerated cellulose with an exclusion limit of 1 KDa. The concentration of proteins is continued until a concentration of 10 mg/ml total protein is reached in retentate (protein concentration is controlled by biuret reaction). Approximately 20 ml of protein solution resulting after concentration are sterilized by filtration on filter of 0.2 µm and are added over the mixture mycelium - plant material, to increase the concentration of proteins of interest, respectively, the cerato-platanins.

The flat bottom glass flask with the mixture mycelium - plant material, enriched in the amphiphile proteins of interest, is incubated at the temperature of 28°C, on a rotary shaker, 70 rpm, for 7 days. After 7 days the mycelium and the rice husk rich in silicon are axenically removed. The mixture is poured into sterile centrifugal bottles of 500 mL. The tubes are centrifuged in an Eppendorf centrifuge 5810 (Eppendorf), with swinging rotor A-4-81, in which the vials are inserted, balanced 2 by 2. The bottles are centrifuged at the speed of 2229 rpm, which corresponds in the case of the swinging rotor A-4-81, with radius of 18 cm, to a relative centrifugal force of 1000 *x* g.

The biomass of the Td50 strain, together with the rice husk is recovered, dried under mild conditions, max. 40°C, and is used as a treatment in agriculture, as a microbial plant biostimulant. Plant biostimulants are a new category of inputs used in plant culture technologies, situated between the fertilizers and the plant protection products. The application of plant biostimulants increases the nutrients uptake and nutrient use efficiency, increases the plant tolerance to abiotic stress and improves the yield quality. In the axenically recovered supernatant, with spores from mycelium, amphiphilic proteins, silica nanoparticles associated with carbohydrates, a 10 mM sodium selenite solution is aseptically added, in a ratio of 10 ml selenite solution to 90 ml supernatant.

The axenic recovered supernatant with selenite is aseptically distributed in Erlenmeyer flasks of 1 liter, each with 200 ml supernatant. The flasks are covered with cotton plugs. The medium is incubated at 28°C, on a rotary shaker, 70 rpm for 7 days. The spores form fungal biomass, which synthesizes selenium nanoparticles. After 7 days the new formed mycelium is separated by tangential ultrafiltration on a Prostak system (Merck Group), fitted with a membrane of 0.45 µm, made from hydrophilic polysulfone. The nanoparticle suspension resulted as permeate is concentrated by tangential ultrafiltration, on a Prostak (Merck Group), fitted with a Ultracel membrane (Merck Group), made from regenerated cellulose, with an exclusion limit of 1 KDa, and the approx. 200 ml of resulted final concentrate are sterilized on a membrane of hydrophilic polyethersulfone (Millipore Express® SHC, Merck Group).

The prepared product is in the form of a red suspension.

Total silicon content and total selenium is determined in samples by ICP-MS (System 7800 ICP-MS, Agilent, Santa Clara, CA, USA). There are determines values of 20.2 ± 4.5 mg/mL Si and 1.05 ± 0.27 mg/ml Si. The distribution of particle size is determined by using the non-invasive technique of background laser light scattering (Zetasizer Nano ZS, Malvern Instruments, Malvern, United Kingdom). A distribution of hybrid nanoparticles with dimension between 72 and 124 nm is be determined. Population of nanoparticles are homogeneous, with a dominant diameter of 92 nm, maximum volume of 27.8%. The stability of nanoparticles was estimated by determining the zeta potential Zeta, through capillary electrophoresis coupled with the measurement of particle mobility by Doppler effect (Zetasizer Nano ZS). There are determined value ranging from -44 ± 9.7 mV, which indicates a good stability of the population of hybrid nanoparticles, silica - zerovalent selenium. This value is similar to that obtained for hybrid nanoparticles of selenium - silica, chemically synthesized and associated by amphiphile molecules of lignosulfonate (Modrzejewska-Sikorska et al. 2017 International Journal of Biological Macromolecules, 103: 403-408).

*Example 2.* The process is the same as in Example 1, with the difference that the strain *Trichoderma asperellum* Td36b which is producing swolenins, deposited under number P (F) 001434 at National Collection of Agricultural and Industrial Microorganisms (NCAIM) Budapest, is used. The plant material is moistened with double distilled water. The incubation of the strain is carried out at 30°C and the incubation time is 5 days. Finally, there are determined 22.4 ± 5.2 mg/mL Si and 0.98 ± 0.23 mg/mL Se, with a distribution of hybrid nanoparticles of a size between 75 and 142 nm. The population of nanoparticles is homogeneous, with a dominant diameter of 98 nm, maximum volume of 22.3%, and the potential Zeta has values of -38 ± 8.2 mV, which indicates good stability.

*Example 3.* The process is the same as in Example 1, with the difference that the strain *Trichoderma* spp. T27, which is producing microbial expansins, is used. Removal of mycelium and plant material is carried out by using an EZ-Fit™ unit (Merck Group), with 0.45 µm membrane. There are determined finally values of 18.2 ± 6.7 mg/mL Si and 0.94 ± 0.28 mg/mL Se, with a distribution of hybrid nanoparticles of a size between 62 and 136 nm. The population of nanoparticles is homogeneous, with a dominant diameter of 94 nm, maximum volume of 24.5%, and the potential Zeta has value of between -36 ± 9.3 mV, which indicates good stability.

*Example 4.* It was tested the ability of hybrid silica-selenium nanoparticles, to protect the test plants against the cadmium toxicity. It was worked with rape, Brassica napus oleifera hybrid, Maximus® PR₄₅D₀₃ (Pioneer Du Pont, Afunati, IIfov, Romania), grown in magenta boxes with Murashige Skoog (MS) mineral medium (MS) mineral, in climatic chamber, at 20 ± 1 °C, a light-dark regime 16/8 hours with a low light intensity (150 µE/m2/s) during light periods. In MS were included concentrations of 0.5 mM CdSO4. There were 8 treatements, untreated control, group treated with cadmium, reference treated with 0.2 mg/L (as sodium selenite), reference treated with 30 mg/L (as silicic acid stabilised with choline), reference treated with 0.2 mg/L Selenium (as sodium selenite), and 30 mg/L (as silicic acid stabilised with choline) and products according Examples 1-3, applied as 2 ml per litre, corresponding to 40 mg Si (as silica nanoparticles) and 2 mg Se (as selenium nanoparticles). The suspensions were done in 1% solutions of sunflower oil ethyl esters of (as spraying adjuvant). There were applied to each 5 days seedlings 50 µL of the tested suspsnsion.. The control was treated with water. Each treatment included 80 seedlings,, distributed in 4 repetitions of 20 young plants (2 Magenta box with 10 seedlings). The repetitions were randomised according to latin square lay-out. After 10 days were determined morphological characteristics of plants (fresh mass of stem and roots), as well as lipid peroxides (as malondialdehide, with thiobarbituric acid (Heath and Parker, 1968, Archives of biochemistry and biophysics, 125:189-198)

**Tab (1). The influence of treatments with products containing nanoparticles suspension and reference products on the toxicity of cadmium to rapeseed (Cv. Maximus® PR₄₅D₀₃)**

| Varianta experimental 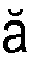 | Fresh weight stem (mg) | Fresh weight roots (mg) | Peroxizi lipidici (MDA, µM/g s.p.) |
|---|---|---|---|
| Untreated control, no Cd | 124±23 | 25±9 | 5,18±0,83 |
| Untreated, +Cd | 22±10 | 10±5 | 8,23±1,64 |
| Ssocium selenite, 0,2 mg/L Se, +Cd | 103±16 | 18±7 | 6,37±0,73 |
| Silicic acid, 30 mg/L Si, +Cd | 110±20 | 23±6 | 6,22±0,82 |
| Sodium selenite, 0,2 mg/L Se + silicic acid, 30 mg/L Si, +Cd | 120±15 | 27±9 | 5,14±0,87 |
| Product Ex.1, +Cd | 144±14 | 37±8 | 4,83±1,08 |
| Product Ex.2, +Cd | 128±19 | 32±12 | 4,23±1,48 |
| Product Ex.3, +Cd | 132±16 | 30±8 | 4,72±1,57 |

Products made in accordance with the examples have a superior efficacy in protecting against toxic effects of cadmium.

## Claims

1. Process for preparing stable and associated suspensions of zerovalent selenium and silica nanoparticles, **characterized in that** includes the following steps: cultivation of microbial strains with a plant biostimulant action for plants, highly producing amphiphile proteins with affinity for carbohydrates, on a mediums that promote the induction of amphiphile proteins genes, for 5 days at 28-30°C, on a rotary shaker, 70 rpm; axenic separation of the plant biostimulants fungal mycelium from the supernatant, by centrifugation, at 2500 *x* g; moistening of the biosilica rich plant material with in pure or bi-distilled water, in the proportion of 1 g plant material to 1 ml pure or bi-distilled water; sterilization of moistened biosilica rich plant material, by three repeated cycles, heating at 72 to 75°C for 25-30 min and cooling at room temperature for 6 hours; aseptic adding over biosilica rich plant material, moistened and sterilized, of the plant biostimulants microorganisms wet mycelium, in the ratio of 1 g wet mycelium to 5 g moistened and sterilized plant material and sterilized; bringing over the mixture mycelium - plant material of an sterile minimal mineral medium, in a ratio of 80 ml sterile minimal mineral medium to 20 grams of mixture mycelium - plant material; preparation of a protein solution to be added over the mycelium - plant material, in order to increase the concentration of the amphyphylic proteins by separation of the amphiphile proteins, from the supernatant separately in the first step, and protein purification, concentration and sterilization by filtration; incubation of the mixture mycelium - plant material - protein solution at the temperature of 28 - 30°C, on rotary shaker, 70 rpm for 5-7 days; removal of the mycelium and plant material by centrifuging, at 1000 *x* g or by filtration, on membranes with pores of minimum 0.45 µm; aseptic adding of a 10 mM sodium selenite solution in the supernatant obtained in the previous step and containing spores form from mycelium, amfifile proteins, silica nanoparticles associated with carbohydrates, in a ratio of 10 ml sodium selenite solution to 90 ml supernatant; incubation of the medium with spores formed from mycelium, amphiphile proteins, silica nanoparticles, sodium selenite at 28-30°C, on rotary shaker, 70 rpm, for 5-7 days, followed by separation by tangential ultrafiltration on the membrane of 0.45 µm of the mycelium newly formed; concentration of nanoparticles suspension resulted as permeate through tangential ultrafiltration, followed by its sterilization.

2. Process according to claim 1 **characterized in that** strains of microorganisms with biostimulant action for plants, high producers of amphiphile proteins with affinity for carbohydrates are selected between the strains of Hypocreaceae family, e.g. *Trichoderma harzianum* Td50B, *Trichoderma asperellum* Td36b, *Trichoderma* spp. T27.

3. Process according to claim 1 **characterized in that** the medium which promote the expression of amphiphile proteins with affinity for carbohydrates has the following composition: 10 g/L whey powder with min. 12% protein and min. 72% lactose, 0.68 g/L KH₂PO₄, 0.87 g/L K₂HPO₄, 1.2 g/L (NH₄)₂SO₄, 0.2 g/L KCL, 0.2 g/L CACL₂, 0.2 g/L MgSO₄.7H₂O, 2 mg/L FeSO₄.7H₂O, 2 mg/L MnSO₄.7H₂O and 2 mg/L ZnSO₄.7H₂O.

4. Process according to claim 1 **characterized in that** plant material with high silicon content includes rice husk, *Pleurotus* spent substrate, brewer's spent grain, as well as their mixtures.

5. Process according to claim 1 **characterized in that** the purification of amphiphile proteins produced in the initial media is done by hydrophobic affinity chromatography on a hydrophobic column, and the concentration is done by ultrafiltration.
